# EUROPÄISCHE PATENTSCHRIFT

(11) **EP 3 197 530 B1**
(45) Veröffentlichungstag und Bekanntmachung des Hinweises auf die Patenterteilung: **20.01.2021**
(21) Anmeldenummer: 15783967.1
(22) Anmeldetag: 28.09.2015
(51) Int. Cl.: A61M 25/00, A61M 25/06

(54) **DEHNBARE EINFÜHRSCHLEUSE**
EXPANDABLE INTRODUCER SHEATH
GAINE D'INTRODUCTION EXTENSIBLE

(30) Priorität: 26.09.2014 DE 102014014015
(43) Veröffentlichungstag der Anmeldung: 02.08.2017
(73) Patentinhaber: Bentley InnoMed GmbH, 72379 Hechingen (DE)
(72) Erfinder: OBRADOVIC, Milisav, 79539 Lörrach (DE)
(74) Vertreter: Hoefer & Partner Patentanwälte mbB
(86) Internationale Anmeldenummer: PCT/EP2015/072227
(87) Internationale Veröffentlichungsnummer: WO 2016/046413

(56) Entgegenhaltungen:
- WO-A1-2011/096975
- DE-A1-102008 011 688
- US-A1- 2002 091 355
- US-A1- 2008 300 544
- US-A1- 2011 190 683

## Beschreibung

Die Erfindung betrifft eine Einführschleuse für Katheter in ein Gefäßsystem mit einem Verbindungsventil, einem Einführrohr, das einen proximalen Abschnitt und einen distalen Abschnittaufweist, wobei der distale Abschnitt über einen gegenüber dem proximalen Abschnitt reduzierten Durchmesser verfügt.

Einführschleusen zur Herstellung eines Gefäßzugangs sind bekannt. Sie werden benötigt, wenn für endovaskuläre Behandlungen ein Katheter in ein Gefäßsystem eingeführt werden muss, über den Implantate oder Behandlungsinstrumente in das vaskuläre System eingebracht werden. Solche Einführschleusen bestehen in der Regel aus einem Kunststoff, etwa Polytetrafluorethylen oder anderen fluorierten Polyolefinen. Neben einem Zugang für einen Katheter können derartige Einführschleusen auch weitere Zugänge aufweisen, die beispielsweise zum Einbringen oder Absaugen von Flüssigkeiten verwandt werden können.

Bei endovaskulären Behandlungen ist es häufig erforderlich, nacheinander verschiedene medizinische Vorrichtungen unterschiedlichen Kalibers in das Gefäß einzubringen. Die Einbringung von Mikrokathetern mit einem Führungsdraht ist in der Regel unproblematisch; bei Kathetern mit einem größeren Kaliber, wie sie beispielsweise für die Gefäßdilatation, für das Extrahieren von Thromben oder für das Implantieren von großkalibrigen Stents benötigt werden, stellt dies aber ein Problem dar.

Großkalibrige Einführschleusen oder Einführhilfen sind zum einen schwer zu platzieren und führen zum anderen bei der Entfernung häufig zu Verletzungen der Gefäßwand und des Gewebes im Einführbereich. Als vorteilhaft hat es sich erwiesen, Einführschleusen zu verwenden, die in ihrem Innendurchmesser an den jeweiligen Zweck angepasst werden können, es also auch erlauben, großkalibrige Katheter aufzunehmen, und die sich solchen großkalibrigen Kathetern anpassen können. Es kommt aber zu Problemen, wenn diese Einführschleusen nach der Erweiterung auf den benötigen Durchmesser diesen erweiterten Durchmesser auch nach der Entfernung des Katheters beibehalten.

Aus diesem Grunde wäre es wünschenswert, über eine Einführschleuse zu verfügen, die sich dem jeweils benötigten Katheter anpassen kann, jedoch nach Entfernung des Katheters wieder auf den ursprünglichen Durchmesser reduziert. Eine solche Einführschleuse kann bei Beendigung der Behandlung dann ohne großen Aufwand mit geringer Gefahr weiterer Gefäßverletzungen wieder entfernt werden.

Diese Aufgabe wird mit einer Einführschleuse der eingangsgenannten Art gelöst, bei der das Einführrohr zur reversiblen Veränderung seines Durchmessers längs verlaufende Dehnungselemente mit Einschnitten als Sollbruchstellen aufweist.

Die erfindungsgemäße Einführschleuse für Katheter in ein Gefäßsystem weist ein übliches Verbindungsventil und ein Einführrohr auf. Im Bereich des Verbindungsventils können ein oder mehrere weitere Zugänge vorhanden sein, die vorzugsweise über ein Luer-Lock gesichert sind.

Das Einführrohr gliedert sich in einen proximalen Abschnitt und einen distalen Abschnitt, wobei der proximale Abschnitt einen größeren Durchmesser aufweist, als der distale Abschnitt. Der distale Abschnitt hat einen Innendurchmesser, der die Einführung herkömmlicher kleinkalibriger Katheter ermöglicht. Im platzierten Zustand befindet sich die Einführschleuse mit ihrem proximalen Abschnitt im Zugangsbereich während der distale Abschnitt des Einführrohrs, das über die notwendige Flexibilität verfügt, sich voll oder überwiegend im Gefäßsystem befindet. Um eine problemlose Platzierung zu ermöglichen, ist der Übergangsbereich vom proximalen zum distalen Abschnitt des Einführrohrs konisch ausgebildet.

Um nach erfolgter Platzierung der Einführschleuse und Platzierung eines Führungsdrahts im Gefäßsystem auch großkalibrige Katheter einführen zu können, weist das Einführrohr zur reversiblen Veränderung seines Durchmessers mehrere längsverlaufende Einschnitte auf. Diese Einschnitte sind so ausgebildet, dass sie sich an den größere Kaliber eines Katheters anpassen können, nach Entfernung des Katheters aber wieder ihren ursprünglichen reduzierten Durchmesser annehmen. Im expandierten Zustand kann das Einführrohr einen um 200 % oder mehr erweiterten Durchmesser, im Vergleich zum reduzierten Zustand, annehmen.

Die Einschnitte sind so gestaltet, dass sie bei Einführung eines Katheters mit größerem Kaliber aufreißen, also Sollbruchstellen darstellen. Dazu erstrecken sich die Einschnitte vorzugsweise über etwa 25 bis 95 % der Wandstärke des Einführrohrs. Die Einschnitte können innen oder außen verlaufen, sind vorzugsweise aber außen angeordnet. In der Regel ist eine Einschnitt-Tiefe ausreichend, die etwa 60 bis 90 % der Wandstärke ausmacht.

Die erfindungsgemäße Einführschleuse weist die Dehnungselemente insbesondere im distalen Abschnitt auf. Die Dehnungselemente erfassen in jedem Fall auch den vorzugsweise konisch ausgebildeten Übergangsbereich zwischen proximalem und distalem Abschnitt. Die Dehnungselemente sind dabei gleichmäßig über den Umfang des Einführrohrs verteilt.

Gemäß einer Ausführungsform sind die Dehnungselemente als Einschnitte ausgebildet, die sich jedoch nicht über die gesamte Länge des distalen Abschnitts des Einführrohrs erstrecken.

Insbesondere weist das Einführrohr zwei Arten von Einschnitten auf, von denen erste Einschnitte von proximal nach distal verlaufen und vor dem distalen Ende des Einführrohrs enden, und die zweiten Einschnitte vom distalen Ende nach proximal verlaufen und sich lediglich über einen distalen Bereich des Einführrohrs erstrecken. Bei den zweiten Einschnitten kann es sich um durch die Wandung hindurch reichende Schlitze handeln.

Der distale Abschnitt des Einführrohrs ist damit unterteilt in einen proximalen Bereich und einen distalen Bereich, wobei der distale Bereich den Bereich bezeichnet, in dem die zweiten Einschnitte verlaufen und der proximale Bereich den Bereich, der sich proximal daran bis zum proximalen Abschnitt des Einführrohrs anschließt. Die ersten Einschnitte, soweit sie ebenfalls im distalen Bereich verlaufen, sind vorzugsweise ebenfalls als Schlitze ausgebildet.

Die ersten und die zweiten Einschnitte sind im distalen Bereich des Einführrohrs alternierend angeordnet. Bei dieser Anordnung können die zweiten Schlitze im distalen Bereich des Einführrohrs im expandierten Zustand einen umlaufenden Kranz von mäandrierenden Stegen ausbilden, vergleichbar mit dem mäandrierenden Verlauf von Stegen in einem Stent im expandierten Zustand.

Es ist zweckmäßig, die ersten Einschnitte in etwa der Höhe des Endes der zweiten Einschnitte, d.h. dort, wo der distale Bereich des Einführrohrs beginnt, mit einer Durchbrechung zu versehen, die in etwa das Aussehen einer länglichen Rautenform hat. Ziel dieser Durchbrechung ist es, die Expansion des Einführrohrs zu erleichtern.

Gemäß einer weiteren Variante der Erfindung sind die Dehnungselemente Einschnitte in die Oberfläche des Einführrohrs, die alternierend innen und außen längs verlaufen. Die Einschnitte reichen in diesem Fall über 50 bis 80 % der Wandstärke des Einführrohrs, vorzugsweise über etwa 65 bis 75 %. Diese Form der Einschnitte ermöglicht eine Ziehharmonika-ähnliche Erweiterung des Einführrohrs, wenn ein großkalibriger Katheter hindurch geschoben wird. Der Grad der Erweiterung hängt von der Wandstärke, der Tiefe der Einschnitte und der Dichte der Einschnitte ab.

Derartige Einschnitte können sich über die gesamte Länge des distalen Abschnitts des Einführrohrs erstrecken. Gemäß einer Alternative erstrecken sich die Einschnitte aber nur über den proximalen Bereich des Einführrohrs, während im distalen Bereich die bereits vorstehend beschriebene Schlitzanordnung zur Anwendung kommt, bei der die Schlitze im expandierten Zustand des Einführrohrs einen umlaufenden Kranz von mäandrierenden Stegen ausbilden. Die Zahl der Einschnitte im proximalen Bereich des Einführrohrs kann dabei deutlich höher sein, als die Zahl der Schlitze im distalen Bereich.

In einer weiteren Variante der Erfindung sind die Einschnitte der Dehnungselemente auch im proximalen Bereich des Einführrohrs Schlitze, die durch die Wand des Einführrohrs hindurch reichen. Auch in diesem Fall kann das Einführrohr bei Einführung eines großkalibrigen Katheters entsprechend ausgeweitet werden. Bei dieser Variante muss aber bei der Einführung der Einführschleuse in das Gefäßsystem für eine adäquate Abdichtung im Einführstadium gesorgt werden, da diese Schlitze keine wirksame Abdichtung mit sich bringen

Die Erfindung wird durch die beiliegenden Abbildungen näher erläutert. Es zeigen:
- Fig. 1:: Eine erfindungsgemäße Einführschleuse in nicht expandiertem Zustand;
- Fig. 2:: die Einführschleuse von Figur 1 in expandiertem Zustand;
- Fig. 3:: den distalen Bereich des Einführrohrs in nicht expandiertem Zustand;
- Fig. 4:: den distalen Bereich des Einführrohrs in expandiertem Zustand;
- Fig. 5:: das Einführrohr in expandiertem Zustand mit eingeführtem Katheter.

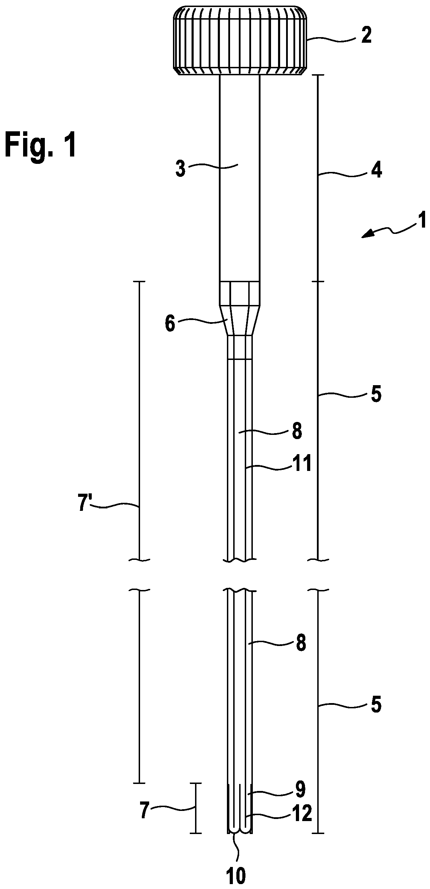

Figur 1 zeigt eine erfindungsgemäße Einführschleuse 1 mit dem Verbindungsventil 2 und dem Einführrohr 3. Das Einführrohr 3 selbst ist unterteilt in einen proximalen Abschnitt 4 mit feststehendem Durchmesser und einem distalen Abschnitt 5, der über Dehnungselemente einen variablen Durchmesser hat. Der distale Abschnitt 5 des Einführrohrs 3 ist wiederum unterteilt in einen distalen Bereich 7 und einen darüber befindlichen proximalen Bereich 7', der an den proximalen Abschnitt 4 heranreicht. Proximaler und distaler Bereich des Einführrohrs ergänzen sich damit zum distalen Abschnitt 5.

Im distalen Abschnitt des Einführrohrs befinden sich längs verlaufenden Einschnitte 11, die ihrerseits Stege 8 aufweisen. Die Einschnitte 11 sind in regelmäßigen Abständen um den Umfang des Einführrohrs 3 verteilt. Die Einschnitte 11 erfassen auch den Übergangsbereich 6, der konisch ausgebildet ist. Im Übergangsbereich 6, der zum distalen Abschnitt 5 gezählt wird, reduziert sich der innere und äußere Durchmesser des proximalen Abschnitt 4 auf den des distalen Bereichs 5.

Die Einschnitte 11 bilden die ersten Einschnitte, die sich vom proximalen Ende des distalen Abschnitts 5 bis kurz vor das distale Ende des distalen Abschnitts 5 erstrecken. Im distalen Bereich 7 sind die Einschnitte 11 vorzugsweise als Schlitze 11' ausgebildet. Im distalen Bereich 7 finden sich zwischen den ersten Einschnitten 11 und 11' zweite Einschnitte 12, ebenfalls regelmäßig angeordnet, die vom distalen Ende 10 über den gesamten distalen Bereich 7 verlaufen und ebenfalls als Schlitze ausgebildet sind. Die alternierende Anordnung der ersten und zweiten Einschnitte im distalen Bereich 7 erlaubt die Bildung eines Kranzes mäandrierender Stege, der den gesamten distalen Bereich 7 einnimmt. Am distalen Ende 10 des Einführrohrs 3 sind die Stege atraumatisch gerundet.

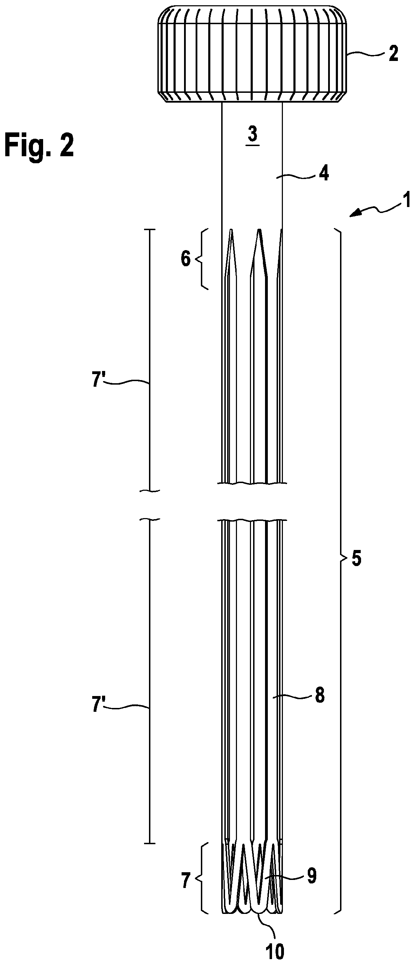

Der expandierte Zustand der Einführschleuse 1 gemäß Figur 1 ist in Figur 2 dargestellt. Die Einschnitte 11, 11' und 12 zwischen den Stegen 8 und 9 sind erweitert und verlaufen bis kurz vor das distale Ende 10 des Einführrohrs 3. Im distalen Bereich 7 des Einführrohrs 3 ist die Stegstruktur zu einem mäandrierenden Stegkranz erweitert; die einzelnen Stege zwischen den zweiten Einschnitten 12 sind V-förmig ausgestellt. Im Übergangsbereich 6 des Einführrohrs 3 verengen sich die erweiterten Einschnitte 11, bis der reguläre Durchmesser des proximalen Abschnitts 4 erreicht ist.

Während in nicht expandiertem Zustand, wie in Fig. 1 dargestellt ist, die Einschnitte 11 im proximalen Bereich 7' noch nicht durch die Rohrwandung hindurch reichen, sind im expandierten Zustand der Fig. 2 die Einschnitte 11 aufgebrochen und zu länglichen Zwischenräumen erweitert. Die Einschnitte 11 reißen bei Einführungen eines großkalibrigen Katheters auf. Im distalen Bereich 7 weiten sich die Schlitze 11' und 12 zu V-förmigen Zwischenräumen zwischen den Stegen 9, die den Stegkranz ausbilden.

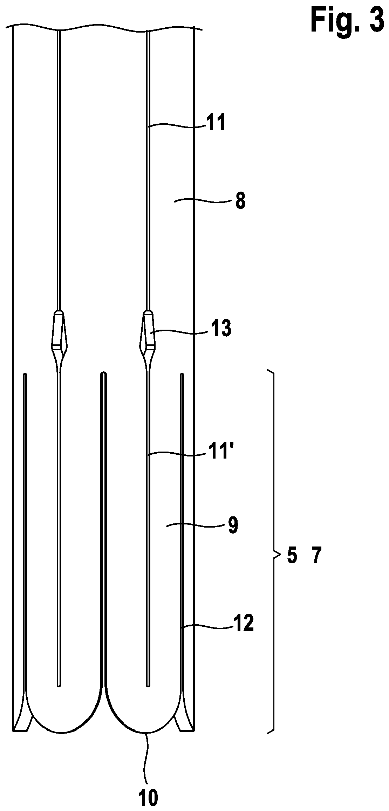

Figur 3 zeigt eine Detailansicht des Einführrohrs 3 mit dem distalen Bereich 7 und dem sich proximal anschließenden Teil des proximalen Bereichs 7', den Einschnitten 11, 11' und 12 sowie den längsverlaufenden Stegen 8 und den im expandierten Zustand mäandrierenden Stegen 9. Zu erkennen sind die in etwa rautenförmigen Erweiterungen 13 der ersten Einschnitte 11 im Bereich des Übergangs in den distalen Bereich 7 wie auch die Stegrundungen am distalen Ende 10 des Einführrohrs 3. Es ist festzuhalten, dass die Einschnitte 11' und 12 als durch die Wandung reichende Schlitze ausgebildet sind.

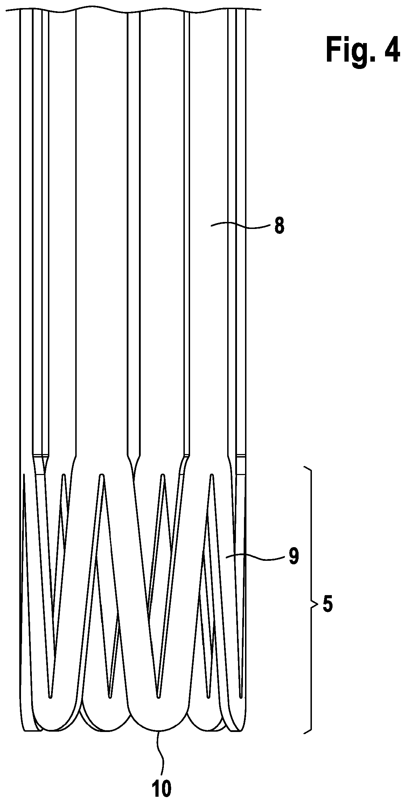

Figur 4 zeigt den Ausschnitt von Figur 3 in expandiertem Zustand mit dem mäandrierenden Stegkranz im distalen Bereich 5 und den parallel verlaufenden Stegen im sich proximal daran anschließenden Bereich. Die Einschnitte 11, 11' und 12 sind zu relativ breiten Zwischenräumen aufgeweitet.

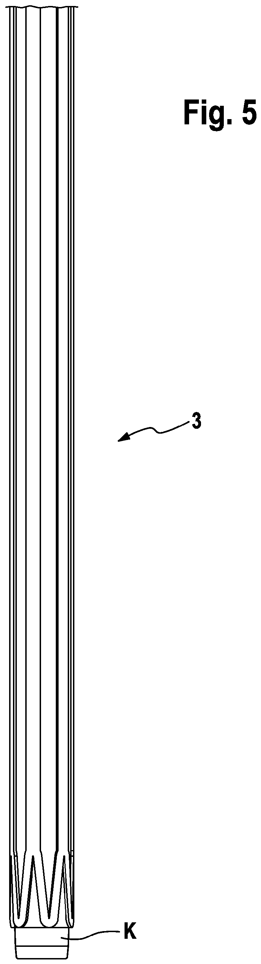

Figur 5 zeigt schließlich den expandierten Ausschnitt des Einführrohrs von Figur 4 mit eingeführtem Katheter K, der die Expansion bewirkt.

Nach Rückzug des Katheters am Ende der Behandlung wird der Expansionsdruck aus dem Einführrohr genommen und das Einführrohr zieht sich wieder auf seinen ursprünglichen Durchmesser zusammen. Dies bewirkt, dass es mit geringem Aufwand aus seiner Position im Gewebe herausgezogen werden kann, ohne dass Gefahr besteht, dass es bei der Entfernung zu Rissen im Gewebe und in der Gefäßwand kommt.

## Patentansprüche

1. Einführschleuse für Katheter in ein Gefäßsystem mit einem Verbindungsventil (2) und einem Einführrohr (3), das einen proximalen Abschnitt (4) und einen distalen Abschnitt (5) aufweist, wobei der distale Abschnitt (5) einen gegenüber dem proximalen Abschnitt (4) reduzierten Durchmesser hat, **dadurch gekennzeichnet, dass** das Einführrohr (3) zur reversiblen Veränderung seines Durchmessers längsverlaufene Dehnungselemente mit Einschnitten (11, 11, 12) als Sollbruchstellen aufweist.

2. Einführschleuse nach Anspruch 1, **gekennzeichnet durch** Einschnitte (11, 11', 12) im distalem Abschnitt (5) des Einführrohres (3).

3. Einführschleuse nach Anspruch 1 oder 2, **dadurch gekennzeichnet, dass** die Einschnitte (11, 11', 12) gleichmäßig über den Umfang des Einführrohres (3) angeordnet sind.

4. Einführschleuse nach einem der vorherstehenden Ansprüche, **dadurch gekennzeichnet, dass** die Einschnitte (11, 11', 12) zumindest teilweise Schlitzform haben.

5. Einführschleuse nach Anspruch 4, **gekennzeichnet durch** erste (11, 11') und zweite (12) Einschnitte im distalem Abschnitt (5) des Einführrohrs (3), von denen die ersten Einschnitte (11, 11') von proximal nach distal verlaufen und die zweiten Einschnitte (12) von distal nach proximal, wobei die zwischen den ersten (11, 11') bzw. zweiten (12) Einschnitten liegenden Bereiche Stege (8, 9) gleicher Breite ausbilden.

6. Einführschleuse nach Anspruch 5, **dadurch gekennzeichnet, dass** die ersten (11') und zweiten (12) Einschnitte in einem distalen Bereich (7) des Einführrohrs (3) alternierend angeordnet sind.

7. Einführschleuse nach einem der Ansprüche 5 oder 6, **dadurch gekennzeichnet, dass** die ersten Einschnitte (11') vor dem distalen Ende (10) des Einführrohrs (3) enden.

8. Einführschleuse nach einem der Ansprüche 5 bis 7, **dadurch gekennzeichnet, dass** die zweiten Einschnitte (12) vom distalen Ende (10) des Einführrohrs (3) ausgehen und in dessen distalen Bereich (7) verlaufen.

9. Einführschleuse nach Anspruch 8, **dadurch gekennzeichnet, dass** die zweiten Einschnitte (12) im expandierten Zustand des Einführrohrs (3) im distalen Bereich (7) einen umlaufenden Kranz von mäandrierenden Stegen (8) ausbilden.

10. Einführschleuse nach einem der Ansprüche 5 bis 9, **dadurch gekennzeichnet, dass** die ersten Einschnitte (11) in Höhe des Übergangs vom proximalen (7') zum distalen (7) Bereich Durchbrechungen der Wandung des Einführungsrohrs (3) aufweisen.

11. Einführschleuse nach einem der Ansprüche 1 bis 3, **dadurch gekennzeichnet, dass** Dehnungselemente Einschnitte (11, 11') sind, die alternierend innen und außen am Einführrohr (3) angeordnet sind.

12. Einführschleuse nach Anspruch 11, **dadurch gekennzeichnet, dass** die Einschnitte über 25 bis 95 %, vorzugsweise 60 bis 90 % der Wandstärke des Einführrohrs (3) reichen.

13. Einführschleuse nach Anspruch 11 oder 12, **dadurch gekennzeichnet, dass** sich die Einschnitte über den proximalen Bereich (7') des Einführrohrs (3) erstrecken.

14. Einführschleuse nach Anspruch 13, **gekennzeichnet durch** erste und zweite Einschnitte (11', 12) im distalen Bereich (7) des Einführrohrs (3), die Stege (9) mit mäandrierendem Verlauf ausbilden.

## Claims

1. An introducer sheath for catheters into a vascular system comprising a connector valve (2) and an insertion tube (3) having a proximal portion (4) and a distal portion (5), wherein the distal portion (5) has a reduced diameter compared to the proximal portion (4),
**characterised in that**,
the insertion tube (3) comprises longitudinally extending expansion elements with incisions (11, 11', 12) as predetermined breaking points for reversibly changing its diameter.

2. The introducer sheath according to claim 1, **characterised by** incisions (11, 11', 12) in the distal portion (5) of the insertion tube (3).

3. The introducer sheath according to claim 1 or 2, **characterised in that** the incisions (11, 11', 12) are uniformly arranged over the circumference of the insertion tube (3).

4. The introducer sheath according to any one of the preceding claims, **characterised in that** the incisions (11, 11', 12) are at least in part slit-shaped.

5. The introducer sheath according to claim 4, **characterised by** first (11, 11') and second (12) incisions in the distal portion (5) of the insertion tube (3), of which the first incisions (11, 11') extend from proximal to distal and the second incisions (12) extend from distal to proximal, wherein the areas located between the first (11, 11') or second (12), resp., incisions form webs (8, 9) of equal width.

6. The introducer sheath according to claim 5, **characterised in that** the first (11') and second (12) incisions in a distal area (7) of the insertion tube (3) are arranged alternatingly.

7. The introducer sheath according to any one of claims 5 or 6, **characterised in that** the first incisions (11') end before the distal end (10) of the insertion tube (3).

8. The introducer sheath according to any one of claims 5 to 7, **characterised in that** the second incisions (12) start from the distal end (10) of the insertion tube (3) and extend in its distal area (7).

9. The introducer sheath according to claim 8, **characterised in that**, in the expanded state of the insertion tube (3), the second incisions (12) form a circumferential ring of meandering webs (8) in the distal area (7).

10. The introducer sheath according to any one of claims 5 to 9, **characterised in that**, at the transition from the proximal (7') to the distal (7) areas, the first incisions (11') have perforations in the wall of the insertion tube (3) .

11. The introducer sheath according to any one of claims 1 to 3, **characterised in that** expansion elements are incisions (11, 11') alternatingly arranged on the inside and outside of the insertion tube (3).

12. The introducer sheath according to claim 11, **characterised in that** the incisions extend across 25 to 95 %, preferably 60 to 90 % of the wall thickness of the insertion tube (3) .

13. The introducer sheath according to claim 11 or 12, **characterised in that** the incisions extend across the proximal area (7') of the insertion tube (3).

14. The introducer sheath according to claim 13, **characterised by** first and second incisions (11', 12) in the distal area (7) of the insertion tube (3), forming webs (9) with a meandering course.

## Revendications

1. Gaine d'introduction pour cathéter dans un système vasculaire avec une soupape de raccordement (2) et un tube d'introduction (3), qui présente une partie proximale (4) et une partie distale (5), dans laquelle la partie distale (5) présente un diamètre réduit par rapport à la partie proximale (4), **caractérisée en ce que** le tube d'introduction (3), pour la modification réversible de son diamètre, présente des éléments de dilatation étendus longitudinalement avec des incisions (11, 11', 12) en tant que points de rupture.

2. Gaine d'introduction selon la revendication 1, **caractérisée par** des incisions (11, 11', 12) dans la partie distale (5) du tube d'introduction (3).

3. Gaine d'introduction selon la revendication 1 ou 2, **caractérisée en ce que** les incisions (11, 11', 12) sont disposées de manière uniforme sur la périphérie du tube d'introduction (3).

4. Gaine d'introduction selon l'une quelconque des revendications précédentes, **caractérisée en ce que** les incisions (11, 11', 12) présentent au moins en partie une forme de fente.

5. Gaine d'introduction selon la revendication 4, **caractérisée par** des premières (11, 11') et deuxièmes (12) incisions dans la partie distale (5) du tube d'introduction (3), parmi lesquelles les premières incisions (11, 11') s'étendent du côté proximal vers le côté distal et les deuxièmes incisions (12) du côté distal vers le côté proximal, dans laquelle les zones situées entre les premières (11, 11') ou deuxièmes (12) incisions réalisent des barrettes (8, 9) de même largeur.

6. Gaine d'introduction selon la revendication 5, **caractérisée en ce que** les premières (11') et deuxièmes (12) incisions sont disposées de manière alternée dans une zone distale (7) du tube d'introduction (3).

7. Gaine d'introduction selon l'une quelconque des revendications 5 ou 6, **caractérisée en ce que** les premières incisions (11') terminent devant l'extrémité distale (10) du tube d'introduction (3).

8. Gaine d'introduction selon l'une quelconque des revendications 5 à 7, **caractérisée en ce que** les deuxièmes incisions (12) partent de l'extrémité distale (10) du tube d'introduction (3) et s'étendent dans la zone distale (7) de celui-ci.

9. Gaine d'introduction selon la revendication 8, **caractérisée en ce que** les deuxièmes incisions (12), dans l'état élargi du tube d'introduction (3), réalisent dans la zone distale (7) une couronne périphérique de barrettes (8) méandreuses.

10. Gaine d'introduction selon l'une quelconque des revendications 5 à 9, **caractérisée en ce que** les premières incisions (11) présentent au niveau de la transition de la zone proximale (7') à la distale (7) des ouvertures de la paroi du tube d'introduction (3).

11. Gaine d'introduction selon l'une quelconque des revendications 1 à 3, **caractérisée en ce que** les éléments de dilatation sont des incisions (11, 11') qui sont disposées sur le tube d'introduction (3) de manière alternée à l'intérieur et à l'extérieur.

12. Gaine d'introduction selon la revendication 11, **caractérisée en ce que** les incisions parviennent sur 25 à 95 %, de préférence 60 à 90 % de l'épaisseur de paroi du tube d'introduction (3).

13. Gaine d'introduction selon la revendication 11 ou 12, **caractérisée en ce que** les incisions s'étendent sur la zone proximale (7') du tube d'introduction (3).

14. Gaine d'introduction selon la revendication 13, **caractérisée par** des premières et deuxièmes incisions (11', 12) dans la zone distale (7) du tube d'introduction (3), qui réalisent des barrettes (9) avec un tracé méandreux.
